# EUROPEAN PATENT APPLICATION

(11) **EP 1 669 051 A1**
(43) Date of publication of application: **14.06.2006**
(21) Application number: 04773159.1
(22) Date of filing: 16.09.2004
(51) Int. Cl.: A61J 1/05, B65D 81/32

(54) **MEDICAL CONTAINER AND ITS USING METHOD**

(30) Priority: 30.09.2003 JP 2003340851
(71) Applicant: NIPRO CORPORATION, Kita-ku, Osaka-shi, Osaka-fu, 531-8510 (JP)
(72) Inventor: Fukushima, Hiroshi c/o Nipro Corporation, Osaka-shi Osaka-fu, 531-8510 (JP); Kataoka, Masaaki c/o Nipro Pharma Corporation, Osaka-shi Osaka 541-0045 (JP)
(74) Representative: Banzer, Hans-Jörg
(86) International application number: PCT/JP2004/013500
(87) International publication number: WO 2005/032451

(57) **Abstract**

Provided are a medical container which shows a display visually recognizable only until a partition wall is separated on a partition wall section of opposed films on outer surfaces of the medical container to display an attention-drawing indicator until immediately before use and deletes the display instantly when the container is opened and brought into a state where partitioned parts communicate with each other, and a method of using the medical container. More particularly, provided is a container which is appropriately used as a multiple-chamber container having a plurality of chambers accommodating a plurality of medicines or the like to be mixed immediately before use, and which can provide a visual indication of whether the separable partition wall has been separated or not so that the condition of the respective chambers communicating with each other can be instantly checked when the multiple-chamber container is used.

## Description

### Technical Field

The present invention relates to a medical container which display an attention-drawing indicator until immediately before use and removes the indicator instantly when the container is opened and brought into a state where partitioned parts communicate with each other, and to a method of using the medical container. More particularly, the invention relates to a medical container which is appropriately used as a multiple-chamber container having a plurality of chambers for containing a plurality of medicines or liquid medicines to be mixed with each other immediately before use, for example, and which can provide a visual indication that the plural chambers are communicating with each other when the medicines or the like are mixed by a person engaged in medical service.

### Background Art

Currently, a medical multiple-chamber container is used to sterilely mix medicines or liquid medicines immediately before use which medicines or liquid medicines cannot be preserved in a mixed condition because of deterioration of quality or other causes. In this container, a plurality of contents are separately stored in a plurality of chambers partitioned by a partition wall or walls which can be easily separated. The partition wall is separated immediately before use by compressing the container using a hand or other methods so that the plural chambers can communicate with each other, and in this condition the contents can be sterilely mixed or dissolved (for example, see Patent Reference No. 1).
However, since a discharge port is formed in one of the plural chambers of the multiple-chamber container, a liquid medicine can be discharged without separating the partition wall and it is thus possible that a liquid medicine is given to a patient without mixing the contents accommodated in the respective chambers.

For overcoming the above drawback, such a container has been developed which is capable of directing attention of a person engaged in medical service to areas around a partition wall when a multiple-chamber container is used (for example, see Patent Reference No. 2). According to this container, a chamber partitioned by the partition wall is provided within a suspension hole of the container. For using the container having the plural chambers, a person engaged in medical service checks the suspension hole to suspend the container. As a necessary consequence, the person simultaneously checks whether the respective chambers are communicating with each other with the partition wall separated.
However, the purpose of development of this multiple-chamber container is to provide a container having only small-capacity medicine chambers which can be brought into the communicating state without fail in particular, and thus the capacity of the chambers to be offered within the small suspension hole is limited.
Additionally, a person engaged in medical service sometimes gives treatment to a plurality of patients simultaneously. In this case, he or she may start other work, temporarily leaving a multiple-chamber container as it is after starting preparation of it for use, and then re-start the preparation. In other cases, another person engaged in medical service may re-start preparation of the container for use. In these cases, there is a possibility that those persons engaged in medical service misunderstand that separation of the partition wall is already completed and start giving a liquid medicine to a patient with the plural chambers not yet communicating with each other. According to the above multiple-chamber container, it is possible to direct attention of a person engaged in medical service to the partition wall, but impossible to promptly provide a visual indication that the partition wall is separated and the respective chambers are communicating with each other.

Patent Reference No. 1: JP-B-6-26563
Patent Reference No. 2: JP-A-2000-5275

### Disclosure of the Invention

### Problems that the Invention is to Solve

Accordingly, it is an object of the invention to provide a medical container which displays an attention-drawing indicator until immediately before use and deletes the indicator instantly when the container is opened and brought into a state where partitioned parts communicate with each other, and to a method of using the medical container. More particularly, it is an object of the invention to provide a container which is appropriately used as a multiple-chamber container having a plurality of chambers for accommodating a plurality of medicines or the like to be mixed immediately before use, for example, and which can provide a visual indication whether a separable partition wall has been separated or not so that the condition of the respective chambers communicating with each other can be instantly checked when the multiple-chamber container is used.

The present inventors have made various detailed investigations for achieving the above object, and found that the object can be attained by a medical container which shows an indicator visually recognizable only until a partition wall is separated on a partition wall section of opposed films.

A medical container according to the invention has a separable partition wall which is formed by heat-welding or bonding parts of container outer members of the medical container opposed to each other. A separation display means capable of displaying an operation for separating the partition wall is provided on a partition wall section of at least one of the container outer members of the medical container. The separation display means can be visually checked from outside the container outer member opposed to the container outer member on which the separation display means is provided prior to separation of the partition wall.

### Advantage of the Invention

In the medical container according to the invention, the separation display means provided on the separable partition wall can be visually recognized from outside the container outer member opposed to the container outer member on which the separation display means is provided prior to use. However, the partition wall section of the container outer members of the medical container is divided and contents accommodated within the medical container come into the space between the container outer members when the partition wall is separated by pressing the container from the outside at the time of use. Thus, a person engaged in medical service can visually recognize that the partition wall has been separated based on the condition that the separation display means cannot be seen. Furthermore, the separation display means according to the invention can promptly provide visual indication whether the partition wall of the medical container has been separated or not even when a person engaged in medical service temporarily leaves the medical container as it is during preparation for use of the container before the person engaged in medical service or another person re-starts the preparation for use.

### Brief Description of the Drawings

[Fig. 1] Fig. 1 is a side view of a multiple-chamber container in an embodiment according to the invention.
[Fig. 2] Fig. 2 is an enlarged cross-sectional view of an example of a separation wall section of the multiple chamber according to the invention.
[Fig. 3] Fig. 3 is an enlarged cross-sectional view of another example of the separation wall section of the multiple chamber according to the invention.
[Fig. 4] Fig. 4 is an enlarged cross-sectional view of still another example of the separation wall section of the multiple chamber according to the invention.
[Fig. 5] Fig. 5 is an explanatory view illustrating an example of separation display means provided on the multiple chamber according to the invention.
[Fig. 6] Fig. 6 is an explanatory view illustrating another example of the separation display means provided on the multiple chamber according to the invention.
[Fig. 7] Fig. 7 is an explanatory view illustrating still another example of the separation display means provided on the multiple chamber according to the invention.

### Description of Reference Numerals and Signs

- 1: multiple-chamber container
- 2: partition wall
- 31, 32: chambers
- 11, 12: container outer members
- 51: separation display means
- 52: symbol display section
- 53: symbol covering section
- 54: base film

### Best Mode for Carrying Out the Invention

A preferred embodiment is hereinafter described in detail with reference to the appended drawings, showing a multiple-chamber container as a medical container according to the invention. The invention is not limited to the multiple-chamber container shown in the embodiment.

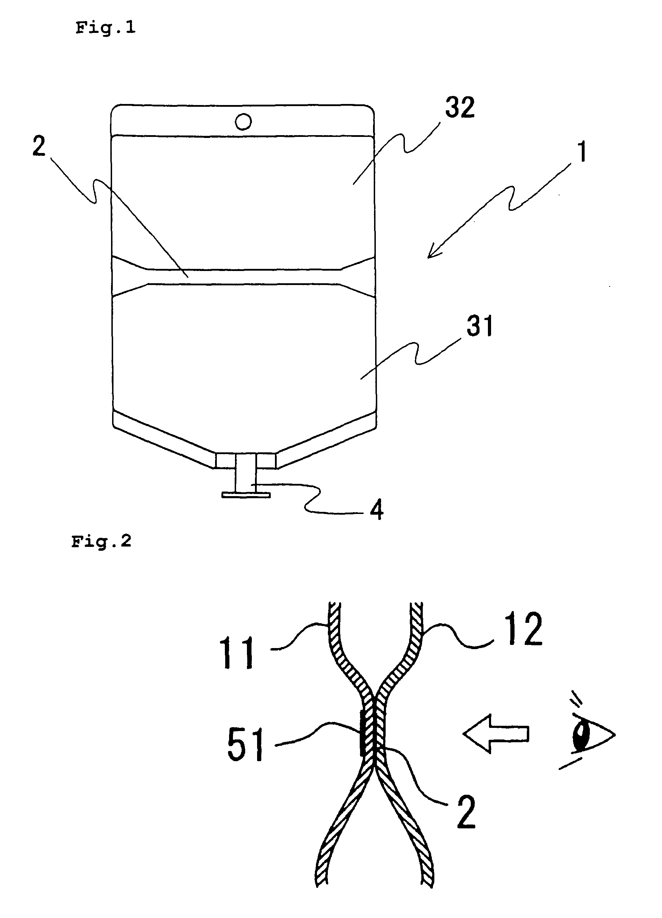

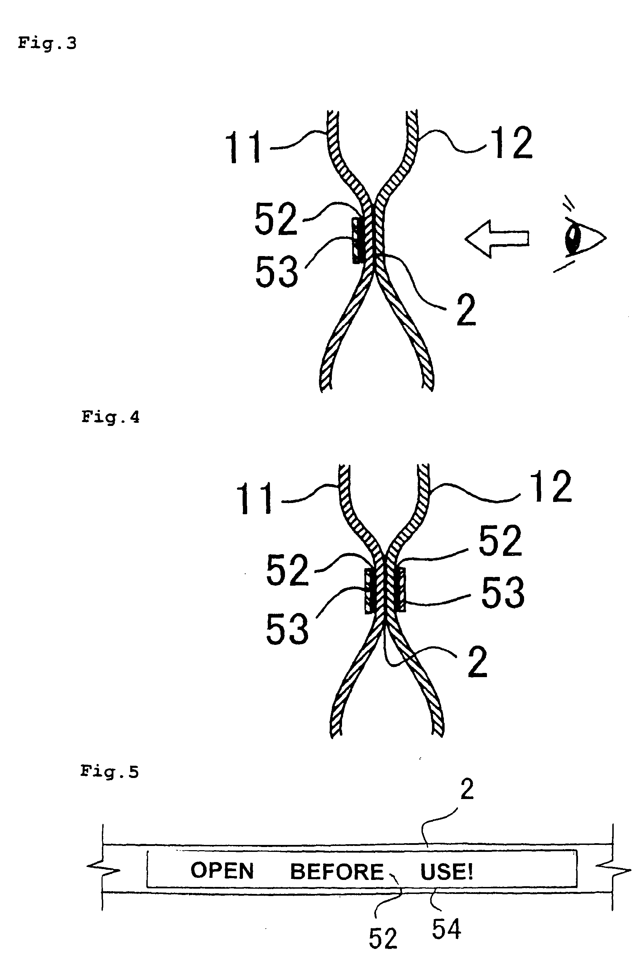

Fig. 1 is a side view of a multiple-chamber container in an embodiment according to the present invention. Fig. 2 is an enlarged cross-sectional view showing an example of a partition wall section of the multiple-chamber container. Figs. 3 and 4 are enlarged cross-sectional views showing other examples of the partition wall section of the multiple-chamber container according to the invention. Figs. 5 through 7 show examples of separation display means provided on the multiple-chamber container according to the invention.

As illustrated in Fig. 1, a multiple-chamber container 1 is partitioned into two chambers 31 and 32 by a separable partition wall 2, for example. The chamber 31 of the two chambers has a discharge port 4 through which medicines or liquid medicines accommodated within the multiple-chamber container are discharged. The number of the chambers formed within the multiple-chamber container 1 may be varied according to the number of the medicines or liquid medicines to be mixed. The number, position and shape of the discharge port may also be varied appropriately according to the types and applications of the liquid medicines to be prepared within the multiple-chamber container.

Container outer members of the multiple-chamber container 1 are made of synthetic resin film. Since one of the features of the invention is that the separation display means can be visually checked from outside the container outer member opposed to the container outer member on which the separation display means is provided, the synthetic resin film is required to be transparent or semi-transparent to such an extent that the separation display means can be visually recognized through the synthetic resin film.

Examples of the synthetic resin used for the synthetic resin film include polyolefin such as polyethylene and polypropylene and substances produced by partially crosslinking these, ethylene-vinyl acetate copolymer, polyester, non-rigid vinyl chloride, and mixtures and copolymers of these. The material of the synthetic resin film may contain not only the synthetic resin mentioned above but also thermal stabilizers, anti-oxidants, ultraviolet absorbers, and others. In addition, surface treatments such as ozone treatment, corona treatment, and vapor deposition treatment may be applied to the material.
The synthetic resin film may be either a single-layer film or multi-layer film. When the synthetic resin film is a single-layer film, the film preferably has a thickness in the range from 5 to 500µm and is formed by T-die molding, inflation molding, hollow molding, or other methods used for manufacturing typical thermal plastic resin. When the synthetic resin film is a multiple-layer film, the film preferably has a thickness in the range of from 10 to 500µm and is formed by co-extrusion molding, dry laminating, extrusion coating, or other methods.

For obtaining the synthetic resin film, the synthetic resin is formed into a pipe-shaped or plate-shaped film by the molding methods described above. When the synthetic resin film is a pipe-shaped film, the film is cut into parts having an appropriate length. When the synthetic resin is a plate-shaped film, two films are overlapped. Then, certain portions of the discrete films opposed to each other are heat-welded to produce a separable partition wall 2. The heat welding is conducted at a higher temperature than the melting point which is lowest in the melting points of the components contained in the innermost layer of the film and also at a lower temperature than the melting point which is highest in the melting points of those components, for example.
When the material of the film is of such a type that cannot produce a separable partition wall, a material capable of producing a separable partition wall is interposed between two films and heat-welded to produce a partition wall.
The edges of the pipe-shaped film or two plate-shaped films having the partition wall 2 are non-separably heat-welded at a higher temperature than the melting point which is highest in the melting points of the components contained in the innermost layer of the film, for example, to produce the multi-chamber container 1. Examples of the heat-welding method include welding using heat metal molds, ultrasonic welding, high frequency welding, and other methods.

Contents are injected through an opening such as the discharge port 4 into the multiple-chamber container 1 produced by the above method prior to or after heat-welding of the edges of the container. The contents may be medicines or liquid medicines, and more specifically dialysis liquids for artificial kidney, substitution liquids for filter-type artificial kidney, transfusion formulations, and other liquids, or powders or solids.
The partition wall 2 of the multiple-chamber container 1 is separated by applying pressure in such a manner as by compressing at least either the chamber 31 or 32 partitioned by the partition wall 2 by hand or by other methods. In the condition where the partition wall 2 is separated, the plural chambers 31 and 32 communicate with each other allowing the contents accommodated within the respective plural chambers to be mixed or dissolved.

In the multiple-chamber container 1 according to the invention, separation display means 51 provided on the partition wall 2 may be provided at the partition wall section of at least either a film 11 or a film 12 opposed to each other. For example, the separation display means 51 may be disposed on one side of the partition wall 2 as illustrated in Fig. 2 (separation display means 51 on the left side in the figure), or may be disposed on both sides of the partition wall 2.
The separation display means 51 can be visually recognized in the direction shown by an arrow in Fig. 2 from outside the film 12 opposed to the film 11 on which the separation display means 51 is provided. When the partition wall 2 is separated to mix or dissolve the contents accommodated within the vertically partitioned chambers 31 and 32 shown in Fig. 1, the partition wall section of the films 11 and 12 is divided and medicines or liquid medicines come into the space between the films. In this or other condition, the separation display means 51 which could be visually checked prior to separation of the partition wall 2 cannot be clearly recognized with the eyes. Accordingly, the person engaged in medical service can easily and visually make sure that the partition wall 2 of the multiple-chamber container 1 is separated and that the plural chambers communicate with each other even when the container 1 is placed, suspended, or disposed in any other condition.

As illustrated in Fig. 3, the separation display means according to the invention may be constituted by a symbol display section 52 as means capable of displaying the separating operation similar to the separation display means 51 and a symbol covering section 53 provided outside the symbol display section 52. By providing the symbol covering section 53, the symbol display section 52 cannot be seen from outside the film 11 on which the symbol display section 52 is disposed but can be seen only in its visual check direction shown by an arrow in Fig. 3, that is, only from outside the film 12 opposed to the film 11 on which the symbol display section 52 is disposed. Generally, product names of medicine sets, prescriptions and the like are shown on the front side of multiple-chamber containers (film 12 side in Fig. 3) so as to clearly indicate their front side at all times. However, the structure including the symbol covering section 53 is more preferable since the symbol covering section 53 prevents erroneous checking of the symbol display section 52 from the side opposite to the correct checking side.

Alternatively, as illustrated in Fig. 4, the separation display means constituted by the symbol display section 52 and the symbol covering section 53 may be provided on both the film 11 and the film 12 opposed to each other. In this case, the means capable of displaying the operation for separating the partition wall 2 can be visually checked from both the front side and the rear side of the multiple-chamber container 1. This structure is more preferable since the condition of the operation for separating the partition wall 2 can be shown on both the front face and the back face of the multiple-chamber container 1.

The symbol display section 52 may be appropriately provided as a sheet element in the form of characters of "OPEN" or an arrow mark, for example, which is described below, or by affixing a figure or the like attracting attention on the outside of the film constituting the partition wall 2, by directly printing the figure or the like on the outer surface of the film, or by other methods. The symbol covering section 53 is preferably provided as a sheet element in the form of a figure as mentioned above produced by methods such as printing, applying, and coating, or by directly printing the symbol display section 52 on the outer surface of the film such that the symbol covering section 53 covers the symbol display section 52.

The symbol display section 52 is required to be visually recognized through the films 11 and 12 constituting the transparent or semi-transparent partition wall 2. Additionally, the symbol display section 52 is required to provide a visual indication of whether the extremely important operation for medicine prescriptions, which operation is to cause the respective chambers containing the medicines or the like to communicate with each other and to mix or dissolve the medicines or liquid medicines, has been carried out or not. It is therefore desired to provide a symbol display section capable of strongly drawing attention of the operator such as the person engaged in medical service who carries out the operation, and it is thus preferable that the color of the characters or figures to be displayed or the color of the background of those characters or figures appeals to the eyes. More specifically, the basic color of the symbol display section 52 itself or its background is preferably a color in the range of Y to RP in the hue circle specified by the Munsell color system, and more preferably a color which belongs to R in the hue specified by the Munsell color system since this color provides caution or warning information more effectively. The symbol covering section 53 for covering only one side of the symbol display section 52 contains non-light-transmissive pigmented ink or the like. The symbol covering section 53 is preferably less conspicuous to the eyes. More specifically, the color of the symbol covering section 53 is preferably a light color containing so-called white color having a bright value in the range from 10 to 8.5 in the Munsell color system.

The area of the portion on which the symbol display section 52 and the symbol covering section 53 are provided is not particularly limited as long as that area is smaller than the area of the partition wall section of the opposed films. However, the area of the portion is preferably large enough that the operator such as the person engaged in medical service can visually recognize the symbol display section 52 without fail.
The symbol display section 52 and the symbol covering section 53 are printed by gravure printing, offset printing, hot stamping, ink jet printing, or other printing methods used for conventional films and containers. The sheet-shaped symbol display section and the film on which the symbol display section is printed are affixed by heat welding, bonding using adhesive, or other known affixing methods.

Next, specific examples of the separation display means according to the invention are described with reference to Figs. 5 through 7.
In an example illustrated in Fig. 5, a transparent base film 54 on which a display of "OPEN BEFORE USE!" as the symbol display section 52 is printed is affixed at the partition wall section on one of the two films constituting the partition wall 2 of the multiple-chamber container 1 described above in detail. The symbol display section 52 may be disposed on either the surface of the base film 54 to be affixed to the partition wall section or on the other surface of the base film 54. The symbol display section 52 is visually checked from outside the film which is one of the two films constituting the partition wall 2 and opposed to the other film to which the base film 54 is affixed. The color of the characters of the symbol display section 52 is preferably a color which appeals to the eyes such as one belonging to R in the hue specified by the Munsell color system described above in detail. It is more preferable to select a color which appeals to the eyes as the color of the entire base film 54 and white as the color of the characters or figures of the symbol display section 52 so as to provide information such as caution and warning with great impact.

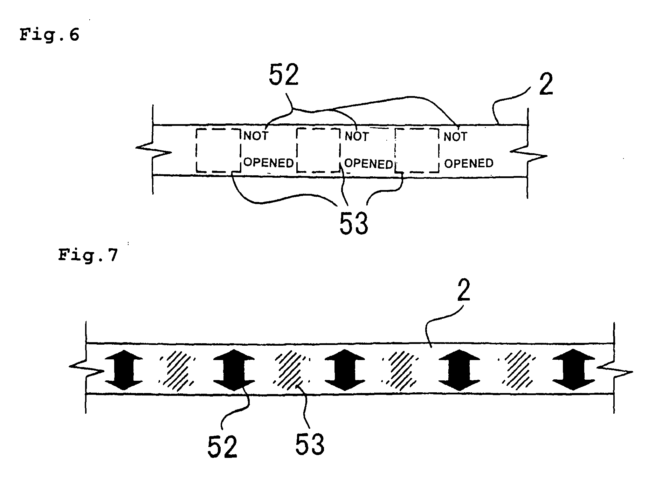

In an example shown in Fig. 6, the symbol display section 52 and the symbol covering section 53 are directly printed on the outer surfaces of both the two films constituting the partition wall 2 of the multiple-chamber container 1. The characters of "NOT" and "OPENED" as the symbol display section 52 shown in the figure are displayed on one of the films with spaces interposed between the characters, and the symbol covering section 53 for covering quadrangular areas of the symbol display section 52 is provided outside the symbol display section 52 such that the symbol display section 52 can be blocked by the symbol covering section 53. On the other hand, the symbol display section 52 and the symbol covering section 53 are disposed on the other film such that the positions of the characters on one film alternate with the positions of the characters on the other film. In this arrangement, the characters of the symbol display section 52 provided on the film on the other side can be visually checked when the partition wall section is seen from this side in the figure, but the characters provided on the film on this side cannot be visually checked since these characters are covered by the symbol covering section 53. Thus, after the partition wall 2 is separated, the symbol display section 52 cannot be seen from outside the films on both sides since both the films are separated and the contents in the container come into the space between the films.

Since the characters of "NOT" and "OPENED" of the symbol display section 52 are important indications for notifying that the operation is not yet performed, it is preferable that a color appealing to the eyes is selected as the color of the characters so as to strongly draw attention of the operator such as the person engaged in medical service. The symbol covering section 53 is produced by coating the symbol covering section 53 by such methods as painting and printing of non-light-transmissive pigmented ink, or by affixing a tape on the symbol covering section 53 for the purpose of coating the symbol display section 52.
The symbols of the symbol display section 52 are not required to be written in Japanese as in the above examples, but may be written in English such as "OPEN HERE!". Alternatively, the symbols may be not only characters but also figures such as arrows and dotted lines as long as they can provide a warning indication that the partition wall is not yet separated and promptly show the condition that the plural chambers of the multiple-chamber container 1 do not yet communicate with each other to the eyes.

Fig. 7 shows an example of the separation display means constituted by figures. The symbol display section 52 and the symbol covering section 53 in this example are directly printed on the outer surfaces of both the two films constituting the partition wall 2 of the multiple-chamber container 1, similarly to the example shown in Fig. 6. Arrows having a color which appeals to the eyes as the symbol display section 52 are displayed at certain intervals on one of the films, and arrows as the symbol covering section 53 which have a light color containing white are provided outside the symbol display section 52 such that the symbol display section 52 is blocked by the symbol covering section 53. On the other hand, the separation display means constituted by the symbol display section 52 and the symbol covering section 53 is disposed on the other film such that the position of the separation display means on one film alternates with the position of the separation display means on the other film. In this arrangement, the symbols of the symbol display section 52 provided on the film on the other side can be visually checked when the partition wall section is seen from this side in the figure, but the symbol display section 52 provided on the film on this side cannot be visually checked since the symbols are covered by the symbol covering section 53. Thus, after the partition wall 2 is separated, the symbol display section 52 cannot be seen from outside the films on either side since both films are separated and the contents of the container come into the space between the films. Accordingly, the condition that the partition wall 2 is separated can be visually checked with ease.

Next, a method of using the multiple-chamber container described above as the medical container according to the invention is described.
The outer members of the multiple-chamber container are made of synthetic resin film. The multiple-chamber container has plural chambers partitioned by the separable partition wall produced by heat-welding opposed parts of the films. The display means capable of showing the operation for separating the partition wall is provided on the outer surface of the partition wall of the multiple-chamber container. The separation display means on the partition wall can be visually checked from outside the film opposed to the film on which the separation display means is provided. For use of the multiple-chamber container, the partition wall section on the opposed films is divided by separating the partition wall provided on the multiple-chamber container in the condition where the separation display means can be seen. Then, the plural chambers are brought into the state where they can communicate with each other so that the contents accommodated within the respective chambers can be mixed or dissolved. In this condition, the separation display means cannot be seen because of the interruption of the contents. Accordingly, the condition where the partition wall is separated can be visually recognized.

Described in detail is the embodiment where the medical container according to the invention is a multiple-chamber container. However, the medical container according to the invention is not limited to a multiple-chamber container but also a single-chamber container having one chamber for accommodating contents. In the case of the single-chamber medical container, the partition wall 2 is used to seal the medical container. In this case, separation display means on the partition wall is similar to the separation display means provided on the multiple-chamber container, and similar advantages can be offered by the separation display means. The method of using the single-chamber medical container is similar to the method of using the multiple-chamber container except that the steps of mixing or dissolving the contents are not included in the method of using the single-chamber container.

### Industrial Applicability

The invention is appropriately applied to a single-chamber or multiple-chamber container having a separable partition wall or walls. Alternatively, the invention is employed for providing a display (such as "STERILIZED" and "PUT ON GLOVES BEFORE USE! ") which is desired to be informed before opening a bonded or welded portion between an edge and a lid of a main body tray of a package accommodating medical tools such as syringes and needles, for providing a display (such as "SHAKE CONTAINER FOR SUFFICIENT PRIMING BEFORE OPEN!" and "HOLD CONNECTION OF SET FOR TAKING OUT TO AVOID REMOVAL OF CONNECTION" which is desired to be informed before opening an edge seal (all-side seal) of a package for various medical tools accommodating transfusion sets, blood circuits and the like, or for providing a display (such as "USE WITHIN ## HOURS AFTER OPENED" and "AVOID MOISTURE AFTER OPENED AND KEEP IN DRIED CASE" which is desired to be informed before opening a bonded or welded portion of an opening of a bag which is made of resin film material and accommodates medicines or the like.

## Claims

**1.** A medical container having a separable partition wall which is formed by heat-welding or bonding parts of container outer members of the medical container opposed to each other, wherein:
separation display means capable of displaying an operation for separating the partition wall is provided on a partition wall section of at least one of the container outer members of the medical container; and
the separation display means can be visually checked from outside the container outer member opposed to the container outer member on which the separation display means is provided prior to separation of the partition wall.

**2.** A medical container according to claim 1, wherein the container outer members are made of synthetic resin film.

**3.** A medical container according to claim 2, wherein the medical container is a multiple-chamber container which has a separable partition wall formed by heat-welding parts of the opposed films and has a plurality of chambers partitioned by the partition wall.

**4.** A medical container according to any one of claims 1 through 3, wherein:
the separation display means has a symbol display section and a symbol covering section provided outside the symbol display section; and
the symbol covering section covers the symbol display section such that the symbol display section cannot be visually recognized from outside the container outer member on which the separation display means is provided.

**5.** A medical container according to any one of claims 1 through 4, wherein the separation display means is provided on the outer surface of the partition wall section.

**6.** A medical container according to claim 5, wherein the separation display means is printed on the outer surface of the container outer member.

**7.** A medical container according to claim 5, wherein the separation display means is printed on a film attached to the outer surface of the container outer member.

**8.** A method of using a medical container which includes: a separable partition wall formed by heat-welding or bonding parts of opposed container outer members of the medical container; and separation display means capable of displaying an operation for separating the partition wall provided on a partition wall section of at least one of the container outer members of the medical container, the separation display means being visually checked from outside the container outer member opposed to the container outer member on which the separation display means is provided prior to separation of the partition wall, the method being **characterized in that**:
the partition wall section on the opposed container outer members is divided by separating the partition wall provided on the medical container in the condition where the separation display means can be seen from outside the container outer member opposed to the container outer member on which the separation display means is provided; and
separation of the partition wall is visually recognized based on the condition where the separation display means cannot be seen.

**9.** A method of using a medical container which includes a separable partition wall formed by heat-welding or bonding parts of opposed films of the medical container; a plurality of chambers partitioned by the partition wall; and separation display means capable of displaying an operation for separating the partition wall provided on a partition wall section of at least one of the films of the medical container, the separation display means being visually checked from outside the film opposed to the film on which the separation display means is provided prior to separation of the partition wall, the method being **characterized in that**:
the partition wall section on the opposed films is divided by separating the partition wall provided on the medical container in the condition where the separation display means can be seen from outside the film opposed to the film on which the separation display means is provided;
the plural chambers are brought into a state where the chambers can communicate with each other so that a plurality of contents accommodated in the respective chambers inside the container can be mixed or dissolved; and
separation of the partition wall is visually recognized based on the condition where the separation display means cannot be seen by the interruption of the contents.
